# EUROPÄISCHE PATENTANMELDUNG

(11) **EP 2 206 526 A2**
(43) Veröffentlichungstag der Anmeldung: **14.07.2010**
(21) Anmeldenummer: 09175779.9
(22) Anmeldetag: 12.11.2009
(51) Int. Cl.: A61L 31/02, A61L 31/14

(54) **Implantat und Verfahren zur Herstellung desselben**

(30) Priorität: 09.12.2008 DE 102008054400
(71) Anmelder: Biotronik VI Patent AG, 6341 Baar (CH)
(72) Erfinder: Bayer, Ullrich, 18211, Admannshagen-Bargeshagen (DE); Becher, Bärbel, 18057, Rostock (DE); Block, Bernd, 18055, Rostock (DE); Lootz, Daniel, 18119, Rostock (DE)
(74) Vertreter: Lindner-Vogt, Karin L.

(57) **Zusammenfassung**

Die vorliegenden Erfindung beschreibt ein Verfahren zur Herstellung eines Implantats, insbesondere einer intraluminalen Endoprothese, wobei das Grundmaterial des Körpers (5) des Implantats biodegradierbares metallisches Material, vorzugsweise Mg oder eine Mg-Legierung, aufweist. Das Verfahren umfasst die folgenden Schritte:
a) Bereitstellen des Körpers (5) des Implantats,
b) Aufbringen einer ersten Schicht (10) auf mindestens ein Teil der Oberfläche des Körpers (5), welche Ca- und P-lonen enthält, und
c) Aufbringen einer zweiten Schicht (20), welche zumindest teilweise für Ca- und P-Ionen durchlässig ist, derart, dass diese zumindest größtenteils die erste Schicht (10) überdeckt.

Es wird ferner ein entsprechendes Implantat beschrieben, bei dem durch die erfindungsgemäße Herstellung das Degradationsverhalten gesteuert werden kann.

## Beschreibung

Die vorliegende Erfindung betrifft ein Verfahren zur Herstellung eines Implantats, insbesondere einer intraluminalen Endoprothese, wobei das Grundmaterial des Körpers des Implantats biodegradierbares metallisches Material, vorzugsweise Mg oder eine Mg-Legierung, aufweist, sowie ein entsprechendes Implantat.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen, beispielsweise Stents, in der Osteosynthese verwendete Implantate, vorzugsweise Befestigungselemente für Knochen, beispielsweise Schrauben, Platten oder Nägel, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren, zu verstehen.

Heutzutage werden als Implantate besonders häufig Stents eingesetzt, die zur Behandlung von Stenosen (Gefäßverengungen) dienen. Sie weisen als Körper ein rohrförmiges oder hohlzylinderförmiges Grundgitter auf, das an beiden Längsenden offen ist. Das rohrförmige Grundgitter einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderer Implantate kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können.

Ein Ansatzpunkt zur Lösung dieses Problems besteht darin, den Stent bzw. andere Implantate aus einem biodegradierbaren Werkstoff als Grundmaterial zu fertigen.

Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Körper biodegradierbarer Implantate geeignete Werkstoffe können beispielsweise Polymere oder Metalle enthalten. Der Körper kann dabei aus einem oder mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren auf Legierungen von Magnesium, Eisen und/oder Wolfram.

Es sind bereits Stents bekannt, die Beschichtungen mit verschiedenen Funktionen aufweisen. Derartige Beschichtungen dienen beispielsweise der Freisetzung von Medikamenten, der Anordnung eines Röntgenmarkers oder dem Schutz der darunter liegenden Strukturen.

Bei der Realisierung biodegradierbarer Implantate soll die Degradierbarkeit entsprechend der angestrebten Therapie bzw. der Anwendung des jeweiligen Implantats (koronar, intracranial, renal etc.) gesteuert werden. Für einige therapeutische Anwendungen soll das Implantat in einem Zeitraum von mehr als sechs Monaten seine Integrität verlieren.

Hierbei wird unter Integrität, d. h. mechanischer Integrität, die Eigenschaft verstanden, dass das Implantat gegenüber dem undegradierten Implantat kaum mechanische Einbußen besitzt. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, dem Aufhalten des Gefäßes, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers aufweist, oder im Fall eines Stents kaum angebrochene tragende Streben aufweist.

Als für den genannten Zielkorridor der Degradation besonders vielversprechend haben sich biodegradierbare Magnesium-Implantate, insbesondere Magnesium-Stents, erwiesen, die allerdings ihre mechanische Integrität bzw. Stützwirkung einerseits noch zu früh verlieren und andererseits in vitro und in vivo einen stark schwankenden Integritätsverlust aufweisen. Dies bedeutet, dass bei Magnesium-Stents der Kollapsdruck über die Zeit zu schnell verringert bzw. die Verringerung des Kollapsdrucks eine zu große Variabilität aufweist und damit zu unbestimmbar ist.

Im Stand der Technik sind bereits unterschiedliche Mechanismen der Degradationskontrolle von Magnesium-Implantaten beschrieben. Diese basieren beispielsweise auf anorganischen und organischen Schutzschichten oder deren Kombination, die dem humanen Korrosionsmilieu und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem metallischen Material, insbesondere dem metallischen Magnesium beruhen. So wird der Degradationsschutz durch verschieden zusammengesetzte Schutzschichten und durch definierte geometrische Abstände (Diffusionsbarrieren) zwischen dem Korrosionsmedium und dem Magnesiumgrundmaterial abgesichert. Andere Lösungen basieren auf Legierungsbestandteilen des biodegradierbaren Materials des Implantat-Körpers, welche den Korrosionsprozess durch Verschiebung der Lage in der elektrochemischen Spannungsreihe beeinflussen. Weitere Lösungen auf dem Gebiet der gesteuerten Degradation rufen durch das Aufbringen von physikalischen (z.B. lokale Querschnittsveränderungen) und/oder chemische Veränderungen der Stent-Oberfläche (z.B. lokal chemisch unterschiedlich zusammengesetzte Multilayer) Sollbrucheffekte hervor. Mit den zuvor genannten Lösungen gelingt es jedoch meist nicht, die durch den Degradationsprozess eintretende Auflösung und die daraus resultierenden Stegbrüche in das geforderte Zeitfenster zu legen. Die Folge ist eine entweder zu früh oder zu spät einsetzende bzw. eine zu große Variabilität der Degradation des Implantats.

Ein weiteres Problem im Zusammenhang mit Beschichtungen ergibt sich auch daraus, dass Stents oder andere Implantate üblicherweise zwei Zustände annehmen, nämlich einen komprimierten Zustand mit einem kleinen Durchmesser und einen expandierten Zustand mit einem größeren Durchmesser. Im komprimierten Zustand kann das Implantat mittels eines Katheters in das zu stützende Gefäß eingeführt und an der zu behandelnden Stelle positioniert werden. Am Ort der Behandlung wird das Implantat dann beispielsweise mittels eines Ballonkatheters dilatiert bzw. (bei Verwendung einer Formgedächtnislegierung als Implantatmaterial) beispielsweise durch Erwärmung über eine Sprungtemperatur in den expandierten Zustand überführt. Aufgrund dieser Durchmesseränderung ist der Körper des Implantats hierbei einer mechanischen Belastung ausgesetzt. Weitere mechanische Belastungen des Implantats können während der Herstellung oder bei der Bewegung des Implantats im oder mit dem Gefäß, in das das Implantat eingesetzt ist, auftreten. Bei den bekannten Beschichtungen ergibt sich somit der Nachteil, dass die Beschichtung während der Verformung des Implantats reißt (z.B. Bildung von Mikrorissen) oder auch teilweise großflächig entfernt wird. Hierdurch kann eine unspezifizierte lokale Degradation verursacht werden. Außerdem sind das Einsetzen und die Geschwindigkeit der Degradation von der Größe und der Verteilung der durch die Verformung entstehenden Mikrorisse abhängig, die als Fehlstellen schlecht kontrollierbar sind. Dies führt zu einer starken Streuung in den Degradationszeiten.

Aus der Druckschrift DE 10 2006 060 501 ist ein Verfahren zur Herstellung einer korrosionshemmenden Beschichtung auf einem Implantat aus einer biokorrodierbaren Magnesiumlegierung sowie ein nach dem Verfahren erhältliches Implantat bekannt, bei dem nach Bereitstellen des Implantats die Implantatoberfläche mit einer wässrigen oder alkoholischen Konversionslösung enthaltend ein oder mehrere Ionen ausgewählt aus der Gruppe K⁺, Na⁺, NH4⁺, Ca²⁺, Mg²⁺, Z²⁺, Ti⁴⁺, Zr⁴⁺, Ce³⁺, Ce⁴⁺, PO₃³⁻, PO₄³⁻, HPO₄²⁻, H₂PO₄⁻ OH⁻, BO₃³⁻, B₄O₇³⁻, SiO₃²⁻, MnO₄²⁻, MnO₄⁻, VO₃⁻, WO₄²⁻, MoO₄²⁻, TiO₃²⁻, Se²⁻, ZrO₃²⁻ und NbO₄⁻ behandelt wird, wobei eine Konzentration des Ions oder der Ionen jeweils im Bereich von 10⁻² mol/l bis 2 mol/l liegt. Die Behandlung der Implantatoberfläche mit der genannten Konversionslösung bedingt eine anodische Oxidation des Implantats. Sie wird entweder ohne Verwendung einer äußeren Stromquelle (außen stromlos) oder mit einer Stromquelle durchgeführt. Die in dieser Druckschrift beschriebenen Verfahrensbeispiele sowie Elektrolytzusammensetzungen erfüllen jedoch nicht die Erwartungen hinsichtlich Degradationsverhalten und Dilatationsfähigkeit ohne Schichtzerstörung bei der Anwendung für einen Magnesium-Stent.

Aus der Druckschrift US 2008/0086195 A1 ist eine medizinische Vorrichtung wie ein Katheter oder ein Stent bekannt, bei dem eine polymerfreie Beschichtung mittels eines plasmaelektrolytischen Prozesses (plasma electrolytic deposition, PED) aufgebracht wird. Hierbei umfasst die plasmaelektrolytische Beschichtung eine plasmaelektrische Oxidation (plasma electrolytic oxidation, PEO), eine Mikro-Lichtbogenoxidation (micro-arc oxidation, MAO), eine Plasma-Lichtbogenoxidation (plasma-arc oxidation, PAO), eine anodische Funkenoxidation (anodic spark oxidation) oder eine elektrolytische Plasmasättigung (plasma electrolytic saturation, PES). Die plasmaelektrolytische Beschichtung wird mittels gepulster Wechsel- oder Gleichspannung bei Spannungen zwischen -100 V bis 600 V durchgeführt. Die Stromdichten liegen im Bereich von 0,5 bis 30 A/dm². Die bekannte plasmaelektrolytische Beschichtung wird vorgesehen, um zusätzliche Wirkstoffe, welche ein Medikament oder ein anderes therapeutisches Mittel beinhalten, in die Beschichtung einbringen zu können. Diese Druckschrift beschäftigt sich demnach nicht mit dem obigen Problem.

Die Aufgabe der vorliegenden Erfindung besteht somit darin, ein Verfahren zur Herstellung eines Implantats anzugeben, das zu einer Verlängerung der Degradationsdauer des Implantats auf einen Zeitraum größer als fünf Monate führt. Die Aufgabe besteht außerdem darin, ein Implantat mit einer entsprechend langen Degradationsdauer zu schaffen.

Die obige Aufgabe wird durch ein Verfahren gelöst, bei dem die folgenden Schritte durchgeführt werden:
a) Bereitstellen des Körpers des Implantats,
b) Aufbringen einer ersten Schicht, welche Ca- und P-lonen enthält, auf mindestens einen Teil der Oberfläche des Körpers und
c) Aufbringen einer zweiten Schicht, welche zumindest teilweise für Ca- und P-Ionen durchlässig ist, derart, dass diese zumindest größtenteils die erste Schicht überdeckt, d.h. den größten Teil der Oberfläche der ersten Schicht überdeckt.

Hierbei kann die erste Schicht sowohl direkt auf die Oberfläche des Implantatkörpers aufgebracht werden als auch auf eine auf der Oberfläche des Körpers angeordnete Beschichtung (siehe z.B. unten).

Die vorliegende Erfindung nutzt die Erkenntnis, dass die Degradation von metallischen Legierungen, insbesondere von Mg-Legierungen, sowohl beim Einsatz z.B. als vaskuläres Implantat oder als biokorrodierbares Implantat in der Osteosynthese unter Anderem einhergeht mit der Bildung von CaP(Kalziumphosphat)-enthaltenden Korrosionsschichten. Diese können Schichtdicken erreichen, die je nach Verweilzeit im Körper bis zu mehreren Mikrometern betragen und einen hohen Anteil des ehemaligen Anfangsquerschnitts des rein metallischen Implantats aufweisen. Aufgrund ihrer Mikrorissigkeit stellen diese Schichten jedoch selbst kein Korrosionsschutzsystem mit Selbstheilungseigenschaften dar, wie sie z.B. bei in der Technik angewendeten, Cr⁶⁻-Ionen enthaltenden Schutzsystemen beobachtet werden. CaP-Schichten zeigen ein geringes plastisches Verformungsvermögen, das zu einer vergleichsweise schnellen Fragmentierung führt. Die Fragmente lösen sich vom metallischen Grundmaterial und setzen dieses dann erneut dem korrosiven Angriff aus.

Wird nun mittels der erfindungsgemäßen ersten Schicht eine "künstliche" Kalziumphosphat-Schicht erzeugt, so weist diese neben der stöchiometrischen Zusammensetzung auch freie bzw. anders gebundene Ca- und P- Ionen auf. Durch Vorhandensein dieser Ionen und die Durchlässigkeit der zweiten Schicht für diese Ionen kann sich die im Körpermilieu als Folge des Degradationsprozesses entstehende "natürliche" CaP-Schicht durch das Reservoir an freien Ca- und P-lonen schneller ausbilden. Dadurch erfährt die Oberfläche des Implantats einen "Selbstheilungseffekt", der zu einer zusätzlichen Deckschichtbildung führt. Diese Deckschicht stellt somit ein körpereigenes Reaktions/Korrosionsprodukt dar und versiegelt die künstlich erzeugte, darunter liegende CaP-Schicht zumindest über einen begrenzten, vorbestimmbaren Zeitraum. Dies führt zu einer Verschiebung des Integritätsverlustes in einen Zeitraum größer als fünf Monate.

In einem bevorzugten Ausführungsbeispiel wird die erste Schicht mittels eines plasmachemischen Verfahrens oder mittels Sandstrahlen aufgebracht. Die genannten Verfahren eignen sich insbesondere deshalb sehr gut für das erfindungsgemäße Verfahren, da sich durch die Zusammensetzung des Elektrolyten bzw. der durch das Sandstrahlen aufgebrachten Feststoffe die Zusammensetzung der ersten Schicht und damit das Degradationsverhalten gut steuern lässt.

Nach dem Aufbringen der ersten Schicht wird das Implantat vorzugsweise in destilliertem Wasser mehrfach gespült und danach sofort unter Heißluft mit einer Temperatur von etwa 50°C bis etwa 80°C getrocknet.

In einem weiteren bevorzugten Ausführungsbeispiel wird vor dem Aufbringen der ersten Schicht eine dritte Schicht, welche Hydroxide und/oder Oxide und/oder Fluoride des Grundmaterials des Körpers aufweist, aufgebracht. Der Vorteil dieser dritten Schicht besteht darin, dass das gesamte Schichtsystem mit der dritten Schicht eine besonders hohe Schadenstoleranz aufweist. Beispielsweise erzeugen die während des späteren Handlings des Implantats auftretenden mechanischen Belastungen der Stentoberfläche (z.B. Biegebeanspruchung beim Crimpen eines Stents) aufgrund des Vorhandenseins der dritten Schicht keine Risse, die bis zum metallischen Grundmaterial reichen.

Es ist weiterhin von Vorteil, wenn der Körper des Implantats vor dem Aufbringen der ersten Schicht gebeizt wird. Durch die Behandlung mit der Beizlösung, beispielsweise mit 20%-iger alkoholischer Phosphorsäure, die Raumtemperatur aufweist, über einen Zeitraum von etwa 10 Sekunden bis etwa 2 Minuten, werden die Rückstände (Oberflächenkontaminationen) auf dem Implantatkörper bis in eine Tiefe von größer als 2 µm herausgelöst. Das Beizverfahren lässt auch nur sehr wenige atomare Wasserstoffionen, die zu einer Wasserstoff-Versprödung des Grundmaterials führen können, entstehen. Vor dem Aufbringen der ersten Schicht und nach dem Beizen des Implantat-Körpers kann zusätzlich eine Elektropolitur durchgeführt werden.

Die zweite Schicht wird besonders bevorzugt mittels CVD (Chemical Vapor Deposition), PVD (Physical Vapor Deposition) oder Plasmapolymerisation aufgebracht. Diese Verfahren sind deshalb gut für die Herstellung der zweiten Schicht geeignet, weil mit Ihnen besonders dünne Schichten aufgebracht werden können.

Das mit dem erfindungsgemäßen Herstellungsverfahren erzeugte Schichtsystem weist eine hohe Schadenstoleranz, ein hohes plastisches Verformungsvermögen und eine hohe Adhäsion zwischen den einzelnen Schichten und zum Körper auf. Das hohe plastische Verformungsvermögen wird dadurch gewährleistet, dass ggf. entstehende Mikrorisse durch die Porenstruktur der Schichten aufgefangen werden. Die Schichten weisen außerdem eine hohe Biokompatibilität auf, weil sie in ihrer chemischen Zusammensetzung weitestgehend den sich im Körpermilieu bildenden Schichten ähneln. Zudem wird die Entstehung von aus zytotoxischer Sicht problematischer Mengen an Korrosionsprodukten minimiert und es zeigen sich höchstens unkritische inflammatorische Nebenwirkungen im umgebenden biologischen Gewebe.

Die obige Aufgabe wird ferner durch ein Implantat gelöst, das mit einer zumindest auf einem Teil der Oberfläche des Körpers angeordneten ersten Schicht, welche Ca- und P-Ionen enthält, und einer zweiten Schicht, welche zumindest teilweise für Ca- und P-Ionen durchlässig ist, derart versehen ist, dass die zweite Schicht zumindest größtenteils die erste Schicht überdeckt, d.h. den größten Teil der Oberfläche der ersten Schicht überdeckt.

Im Folgenden wird der Degradations-/Korrosionsmechanismus des erfindungsgemäßen Implantats erläutert, der zu einer Degradation im gewünschten Zeitfenster führt. Bei einem derartigen erfindungsgemäßen Implantat sorgt, wie oben erläutert, die unter der zweiten Schicht liegenden ersten Schicht, die eine "künstliche" CaP-Schicht darstellt, nach einer Implantation am Behandlungsort dafür, dass Ca- und P-lonen schnell für den Aufbau einer "natürlich" entstehenden, außen liegenden CaP-Schicht zur Verfügung stehen. Der Austausch von Ca- und P-lonen erfolgt hierbei durch die zweite Schicht hindurch, welche für diese Ionen durchlässig ist. Durch den Austausch der Ca- und P-lonen durch die zweite Schicht wird zunächst eine Iokal begrenzte Selbstheilung der in der zweite Schicht vorhandenen Mikrolöcher erreicht. Nach einem weiteren Voranschreiten der Korrosion findet ein verstärkter lokaler Austausch von Ca- und P-lonen der ersten Schicht und der außen liegenden "natürlichen" CaP-Schicht durch die zweite Schicht hindurch statt. Hierbei befinden sich zunächst die Selbstheilungseffekte und die Korrosion noch im chemischen Gleichgewicht und die Ionen der beiden CaP-Schichten vermischen sich miteinander. Danach beginnt auch die Degradation der ersten Schicht, wobei das Grundmaterial von der Degradation noch nicht betroffen ist. Unterdessen schreitet die Korrosion der zweiten Schicht weiter voran und eine Selbstheilung ist nicht mehr gewährleistet. Die zweite Schicht weist nun zahlreiche Löcher auf, in denen sich die Ca- und P-lonen der ersten Schicht und der "natürlichen" CaP-Schicht vermischt haben. Die Schwachstellen in der ersten Schicht reichen bei weiterem Voranschreiten der Degradation bis zum Grundmaterial des Implantats, dessen Degradation nun ebenfalls einsetzt. Jetzt findet der lonenaustausch über den gesamten Querschnitt des Beschichtungssystems statt und die Degradation wird beschleunigt.

In einem bevorzugten Ausführungsbeispiel enthält die zweite Schicht Parylene. Insbesondere bei einer Schichtdicke zwischen etwa 1 µm und etwa 3 µm weist eine Parylene-Schicht ein besonders vorteilhaftes Verhalten bei der Degradation auf. Besonders im Bereich dieser Schichtdicken ist die Parylene-Schicht durchlässig für Ca- und P-lonen und ist außerdem so flexibel, so dass sie auch eine Dilatation eines Implantats ermöglicht. Besonders bevorzugt wird die Parylene-C Variante eingesetzt, wobei aber auch eine Parylene N enthaltende Beschichtung verwendet werden kann.

In einem weiteren bevorzugten Ausführungsbeispiel beträgt die Schichtdicke der ersten Schicht zwischen etwa 0,5 µm und etwa 10 µm. Der erfindungsgemäße Schichtdickenbereich ist von Vorteil, da er einerseits noch eine wirksame Diffusionsbarriere für das korrosive Medium darstellt und anderseits (durch die Schichtdickenbegrenzung nach oben) noch ein Mindestmaß an plastischem Umformvermögen gewährleistet. Größere Schichtdicken können beim Dilatieren Risse und Schichtdelaminationen hervorrufen.

In einem weiteren bevorzugten Ausführungsbeispiel ist unter der ersten Schicht auf der Körperoberfläche eine dritte Schicht angeordnet, welche Hydroxide und/oder Oxide und/oder Fluoride des Grundmaterials des Körpers aufweist und eine Schichtdicke von vorzugsweise kleiner als etwa 150 nm hat. Die Vorteile der dritten Schicht wurden oben bereits erläutert. Die dritte Schicht kann insbesondere dann entfallen, wenn die Degradationsdauer -je nach Indikation- so eingestellt werden soll, dass sie am unteren Ende der Degradationsdauer des erfindungsgemäßen Implantats liegt.

Das erfindungsgemäße Verfahren bzw. das erfindungsgemäße Implantat werden nachfolgend anhand von Beispielen und Figuren erläutert. Dabei bilden alle beschriebenen und/oder gezeigten Merkmale den Gegenstand der Erfindung, unabhängig von ihrer Zusammenfassung in den Ansprüchen oder deren Rückbeziehung.

Es zeigen schematisch:
- Figur 1: einen Querschnitt durch einen Teil eines Körpers eines ersten Ausführungsbeispiels eines erfindungsgemäßen Implantats und
- Figur 2: einen Querschnitt durch einen Teil eines Körpers eines zweiten Ausführungsbeispiels eines erfindungsgemäßen Implantats.

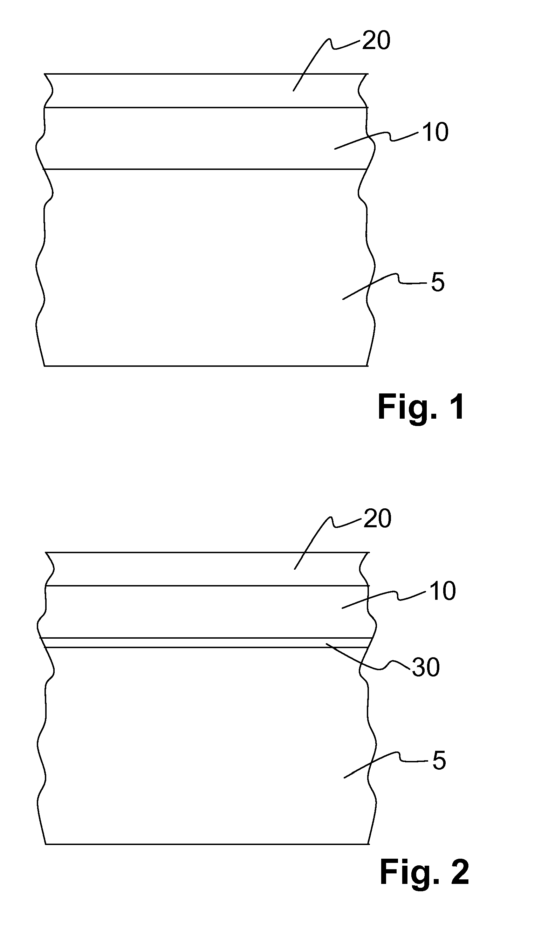

In Figur 1 ist ein Querschnitt eines Teils des Körpers eines ersten Ausführungsbeispiels eines erfindungsgemäßen Implantats dargestellt. Das Implantat ist beispielsweise ein Stent. Auf der Oberfläche des Körpers 5 ist eine erste Schicht 10 angeordnet, welche Ca-Ionen und P-lonen enthält und vorzugsweise eine Schichtdicke zwischen etwa 0,5 µm und etwa 10 µm aufweist.

Die erste Schicht 10 setzt sich beispielsweise aus folgenden Komponenten zusammen: (in abnehmender Reihenfolge)
- Magnesiumphosphat (30 - 50 Masse%)
- Calciumphosphat (30-40 Masse%)
- Magnesiumoxid (15-20 Masse%)
- Magnesiumcarbonat (10-15Masse%)
- Magnesiumhydroxid (10-15Masse%)
- Rest (<5 Masse%): nichtstöchiometrische Kalium- und Natriumverbindungen

Die erste Schicht 10 kann beispielsweise mittels eines plasmachemischen Verfahrens bei Spannungen zwischen 250 und 500 V, Stromdichten zwischen 0,5 und 5 A/dm², Pulsfrequenzen zwischen 100 Hz und 10 kHz und unter Anwendung einer Gegenkathode aus Edelstahl 1.4301 durch Behandlung des Impantat-Grundkörpers in einem wässrigen Elektrolyt der folgenden Zusammensetzung
- 65 ml/l Ethylendiamin-Lösung (99%),
- 80 g/l Kaliumdihydrogenphosphat,
- 20 ml/l wässrige Ammoniumhydroxidlösung (25%)
- 25 g/l Natriumcarbonat
aufgebracht werden.

Über der ersten Schicht 10 liegt eine zweite Schicht 20, welche vorzugsweise aus Parylene C besteht und eine Schichtdicke zwischen etwa 1 µm und etwa 3 µm aufweist.
Die zu behandelnden Stents werden in einer Beschichtungskammer platziert. Mit einer auf das Kammervolumen und die Stentoberfläche berücksichtigenden Einwaage (z. B. ca. 4g bei 1 µm und 10 g bei 3µ Schichtdicke) wird der Beschichtungsprozess gestartet.

Die Verdampfertemperatur beträgt dabei zwischen 100°C und 170°C. Das pulverförmige Monomer wird Aufgrund des angelegten Kammervakuums von ca. 0,5 bis 50 Pa über eine Heizplatte gesaugt. Die Temperatur der Heizplatte beträgt dabei zwischen 650°C und 730°C. Nach einer Beschichtungszeit von ca. 1 Stunde bei 1 µm (3 Stunden bei 3 µm) weisen die Stents eine homogene Bedeckung mit Parylene C auf.

Figur 2 zeigt ein zweites Ausführungsbeispiel eines erfindungsgemäßen Implantats, bei dem gegenüber dem ersten Ausführungsbeispiel zusätzlich unter der ersten Schicht 10 eine dritte Schicht 30 vorgesehen ist, welche Hydroxide und/oder Oxide und/oder Fluoride des Grundmaterials des Körpers 5 aufweist und eine Schichtdicke von vorzugsweise kleiner als etwa 150 nm hat.

Zur Herstellung einer Hydroxide des Grundkörpers enthaltenden dritten Schicht 30 wird der Körper 5 des Implantats wie folgt behandelt:
Eintauchen bei einer Temperatur von 100°C bis 120°C in wässriger NaOH-Lösung (100 g/l bis 200 g/l) über 5 bis 10 Minuten, anschließend 3-fach Spülen in warmem destillierten H₂0 und Lufttrocknung bei einer Temperatur von 50°C bis 80°C.

Alternativ oder zusätzlich kann der Körper 5 mittels einer Oxidation wie folgt behandelt werden, so dass die dritte Schicht 30 Oxide des Grundmaterials des Körpers 5 aufweist:
Einlagerung in einer Vakuumkammer, Evakuieren auf ca. 0,1 mbar und Zünden eines Sauerstoffplasma bei einem Sauerstoffpartialdruck von ca. 1 mbar, Behandlungsdauer 10 Minuten bis 15 Minuten.

Alternativ oder zusätzlich kann der Körper 5 auch wie folgt fluoridiert werden, so dass die dritte Schicht 30 Fluoride des Grundmaterials des Körpers 5 aufweist:
Tauchen des Körpers 5 in 40%-ige HF bei Raumtemperatur über einen Zeitraum von 2 Stunden bis 48 Stunden, anschließend 1-faches Spülen in warmem destillierten H₂0, kurzes Neutralisieren in wässriger NaOH-Lösung (20g NaOH/l) und nachfolgendes 3-faches Spülen in warmem destillierten H₂0 und Lufttrocknung bei einer Temperatur von 50°C bis 80°C

### Bezugszeichenliste:

- 5: Körper des Implantats
- 10: erste Schicht
- 20: zweite Schicht
- 30: dritte Schicht

## Patentansprüche

1. Verfahren zur Herstellung eines Implantats, insbesondere einer intraluminalen Endoprothese, wobei das Grundmaterial des Körpers (5) des Implantats biodegradierbares metallisches Material, vorzugsweise Mg oder eine Mg-Legierung, aufweist, umfassend die folgenden Schritte:
a) Bereitstellen des Körpers (5) des Implantats,
b) Aufbringen einer ersten Schicht (10), welche Ca- und P-lonen enthält, auf mindestens einen Teil der Oberfläche des Körpers und
c) Aufbringen einer zweiten Schicht (20), welche zumindest teilweise für Ca- und P-lonen durchlässig ist, derart, dass diese zumindest größtenteils die erste Schicht (10) überdeckt.

2. Verfahren nach Anspruch 1, **dadurch gekennzeichnet, dass** die erste Schicht (10) mittels eines plasmachemischen Verfahrens oder mittels Sandstrahlen auf den Körper (5) aufgebracht wird.

3. Verfahren nach einem der vorhergehenden Ansprüche, **dadurch gekennzeichnet, dass** vor dem Aufbringen der ersten Schicht (10) auf mindestens einen Teil der Oberfläche des Körpers (5) eine dritte Schicht (30) aufgebracht wird, welche Hydroxide und/oder Oxide und/oder Fluoride des Grundmaterials des Körpers (5) aufweist.

4. Verfahren nach einem der vorhergehenden Ansprüche, **dadurch gekennzeichnet, dass** der Körper (5) des Implantats vor dem Aufbringen der ersten Schicht (10) gebeizt wird.

5. Verfahren nach einem der vorhergehenden Ansprüche, **dadurch gekennzeichnet, dass** die zweite Schicht (20) mittels CVD, PVD oder Plasmapolymerisation aufgebracht wird.

6. Implantat, insbesondere einer intraluminalen Endoprothese, wobei das Grundmaterial des Körpers (5) des Implantats biodegradierbares metallisches Material, vorzugsweise Mg oder eine Mg-Legierung, aufweist, mit einer zumindest auf einem Teil der Oberfläche des Körpers angeordneten ersten Schicht (10), welche Ca- und P-Ionen enthält, und einer zweiten Schicht (20), welche zumindest teilweise für Ca-und P-lonen durchlässig ist, derart, dass diese zumindest größtenteils die erste Schicht (10) überdeckt.

7. Implantat nach Anspruch 6, **dadurch gekennzeichnet, dass** die zweite Schicht (20) Parylene enthält.

8. Implantat nach einem der Ansprüche 6 bis 7, **dadurch gekennzeichnet, dass** die erste Schicht (10) eine Schichtdicke zwischen etwa 0,5 µm und etwa 10 µm aufweist.

9. Implantat nach einem der Ansprüche 6 bis 8, **dadurch gekennzeichnet, dass** die zweite Schicht (20) eine Schichtdicke zwischen etwa 1 µm und etwa 3 µm aufweist.

10. Implantat nach einem der Ansprüche 6 bis 9, **dadurch gekennzeichnet, dass** unter der ersten Schicht (10) auf der Oberfläche des Körpers (5) eine dritte Schicht (30) angeordnet ist, welche Hydroxide und/oder Oxide und/oder Fluoride des Grundmaterials aufweist und eine Schichtdicke von vorzugsweise kleiner als etwa 150 nm hat.
